# EUROPEAN PATENT APPLICATION

(11) **EP 1 607 386 A1**
(43) Date of publication of application: **21.12.2005**
(21) Application number: 04723794.6
(22) Date of filing: 26.03.2004
(51) Int. Cl.: C07D 207/16, A61K 31/401, A61P 3/06, A23L 1/30, A23K 1/16

(54) **LIPID METABOLISM IMPROVING AGENT**

(30) Priority: 26.03.2003 JP 2003084393
(71) Applicant: KYOWA HAKKO KOGYO CO., LTD., Chiyoda-ku, Tokyo 100-8185 (JP)
(72) Inventor: KAMIYA, Toshikazu, c/o Healthcare Research Lab., Tsukuba-shi, Ibaraki 305-0841 (JP); SHIRAI, Akio, c/o Head Office,, Chiyoda-ku, Tokyo 100-8185 (JP)
(74) Representative: Vossius & Partner
(86) International application number: PCT/JP2004/004311
(87) International publication number: WO 2004/085389

(57) **Abstract**

An object of the present invention is to provide a lipid metabolism improving agent, or to provide foods and drinks, food and drink additives, and feeds or feed additives for improving lipid metabolism.

In order to achieve such an object, the present invention provides a lipid metabolism improving agent comprising hydroxyproline, a hydroxyproline derivative or a pharmaceutically acceptable salt thereof or foods and drinks, food and drink additives, feeds or feed additives for improvement of lipid metabolism comprising the same.

## Description

### Technical Field

The present invention relates to a lipid metabolism improving agent and also to foods and drinks, food and drink additives, feeds and feed additives for improving lipid metabolism.

### Background Art

Blood contains the following four kinds of lipids (fats) : cholesterol, phospholipid, triglyceride (neutral fat) and free fatty acid, and they are collectively called serum lipids. The state where an amount of the lipids contained in blood is abnormally high is called hyperlipemia or hyperlipidemia and a drug which is capable of improving such a symptom is called a lipid metabolism improving agent. As a drug used for improvement of hypercholesterolemia or hypertriglyceridemia, HMG-CoA reductase inhibitor, fibrate-type drug, nicotinic acid-type drug, anion-exchange drug, probucol, etc. have been known.

On the other hand, no drug which is used mainly for improvement of hyper-free fatty acidemia has been known. It has been considered that hyper-free fatty acidemia not only shows fat toxicity to pancreatic β cells but also induces inhibition for insulin action in periphery whereby it causes resistance to insulin *(Journal* of *Nippon Medical School,* 2001, volume 68, no. 2, pages 194 to 197).

Hydroxyproline widely occurs in nature as a major amino acid component of collagen and its N-acetyl derivative is used as an anti-inflammatory agent. It has also been used as a material for synthesis of various medicaments such as antibiotic substances of carbapenem type, blood pressure depressant, anti-asthma agent, improving agent for peripheral circulation and blood coagulation inhibitor. Further, due to its functional characteristic of having moisturizing property, it has been used for cosmetics as well *(Bioscience* and *Industry,* 1998, volume 56, no. 1, pages 11 to 16). It has also been used as a food additive for adjustment of quality of taste and improvement in taste of fruit juice, refreshing soft drink and commonly used food or as a material for flavor ("Commentary for Official Formulary of Food Additives", Seventh Edition, published by Hirokawa Shoten, 1998, pages D-1114 to 1115).

With regard to a pharmacological action of hydroxyproline, an action for suppressing aging of the skin and an action for improving skin quality (WO 00/51561; Japanese Published Unexamined Patent Application No. 080321/2002), anti-inflammatory action, anti-rheumatic action, analgesic action and wound-healing action (Japanese Published Unexamined Patent Application No. 337526/1996) have been known, and there has been no report for an action for improving lipid metabolism.

### Disclosure of the Invention

An object of the present invention is to provide a lipid metabolism improving agent as well as foods and drinks, food and drink additives, feeds or feed additives for improving lipid metabolism.

The present invention relates to the following (1) to (11).
(1) A lipid metabolism improving agent, which comprises, as an active ingredient, hydroxyproline or a hydroxyproline derivative represented by the formula (I) [hereinafter referred to as the compound (I)] or a pharmaceutically acceptable salt thereof: 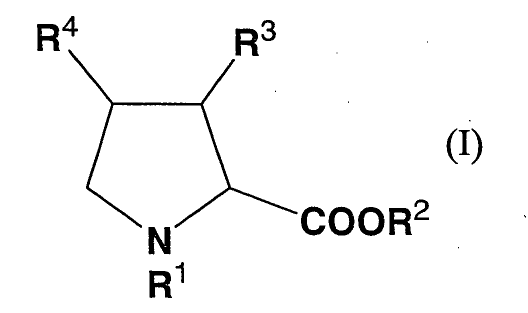 [wherein R¹ is hydrogen or acyl; R² is hydrogen or a saturated or unsaturated hydrocarbon group; and one of R³ and R⁴ is hydrogen while the other is OR⁵ (in which R⁵ is hydrogen or acyl)].
(2) The lipid metabolism improving agent according to (1), wherein the lipid metabolism improvement is intended for improvement of hypertriglyceridemia or a hyper-free fatty acidemia.
(3) A food and drink or food and drink additive for improvement of lipid metabolism, which comprises the compound (I) or a pharmaceutically acceptable salt thereof as an active ingredient.
(4) The food and drink or food and drink additive for improvement of lipid metabolism according to (3), wherein the lipid metabolism improvement is intended for improvement of hypertriglyceridemia or a hyper-free fatty acidemia.
(5) A feed or feed additive for improvement of lipid metabolism, which comprises the compound (I) or a pharmaceutically acceptable salt thereof as an active ingredient.
(6) The feed or feed additive for improvement of lipid metabolism according to (5), wherein the lipid metabolism improvement is intended for improvement of hypertriglyceridemia or a hyper-free fatty acidemia.
(7) A method for improving of lipid metabolism, which comprises administering the compound (I) or a pharmaceutically acceptable salt thereof.
(8) The method according to (7), wherein the lipid metabolism improvement is intended for improvement of hypertriglyceridemia or a hyper-free fatty acidemia.
(9) Use of the compound (I) or a pharmaceutically acceptable salt thereof for the manufacture of a lipid metabolism improving agent.
(10) Use of the compound (I) or a pharmaceutically acceptable salt thereof for the manufacture of foods and drinks or food and drink additives for improvement of lipid metabolism.
(11) Use of the compound (I) or a pharmaceutically acceptable salt thereof for the manufacture of feeds or feed additives for improvement of lipid metabolism.

With regard to the definition for each group in the compound (I), the acyl includes, for example, straight or branched acyl group having 2 to 23 carbon atoms and, specific examples thereof include, for example, acetyl, propionyl, butyryl, isobutyryl, valeryl, hexanoyl, heptanoyl, octanoyl, decanoyl, eicosanoyl, tricosanoyl, etc. Among them, acetyl and propionyl are preferred.

The saturated or unsaturated hydrocarbon group includes, for example, a straight or branched, and saturated or unsaturated hydrocarbon group having 1 to 30 carbon atoms and, specific examples thereof include, for example, methyl, ethyl, propyl, isopropyl, butyl, tert-butyl, isobutyl, 3-methyl-1-butyl, 2-methyl-1-butyl, pentyl, hexyl, octyl, 2-ethylhexyl, lauryl, myristyl, palmityl, stearyl, oleyl, eicosanoyl, phytyl, behenyl, melissyl, triacontyl, etc. Among them, a straight or branched, and saturated or unsaturated hydrocarbon group having 1 to 20 carbon atoms is preferred, and as more specific examples, mention may be made of methyl, ethyl, propyl, isopropyl, butyl, tert-butyl, isobutyl, 3-methyl-1-butyl, 2-methyl-1-butyl, pentyl, hexyl, octyl, 2-ethylhexyl, lauryl, myristyl, palmityl, stearyl, oleyl, eicosyl, phytyl, etc.

The compound (I) in which R¹ and R² are hydrogen and one of R³ and R⁴ is hydrogen while the other is OH, is hydroxyproline. Hydroxyproline widely occurs in nature as a major amino acid component of collagen and as an amino acid component of elastin. It has been known that there exist eight kinds of stereoisomers of natural hydroxyproline which are distinct from one another, depending on whether proline is the D-form or the L-form, whether the hydroxyl group is at the 3-position or the 4-position, and whether the stereoisomer is the cis-form or the trans-form. Specific examples thereof are mentioned as cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

Although hydroxyproline of any such structure is able to be used in the present invention, trans-4-hydroxy-L-proline is preferably used.

Hydroxyproline is able to be produced by subjecting collagen derived from animals such as pig and cow to acid hydrolysis and purifying the hydrolysate according to a conventional method. However, hydroxyproline produced using microorganisms is preferably used.

Useful microorganisms include those belonging to the genus selected from the group consisting of the genus *Amycolatopsis,* the genus *Dactylosporangium* and the genus *Streptomyces* or those into which a proline 3-hydroxylase gene or a proline 4-hydroxylase gene derived from these microorganisms has been introduced. Introduction of a proline 3-hydroxylase gene or a proline 4-hydroxylase gene derived from a microorganism belonging to the genus selected from the group consisting of the genus *Amycolatopsis,* the genus *Dactylosporangium* and the genus *Streptomyces* into a microorganism can be carried out according to the methods described in Molecular Cloning, A Laboratory Manual, Second Edition, Cold Spring Harbor Laboratory Press (1989), Current Protocols in Molecular Biology, John Wiley & Sons (1987-1997), etc.

Furthermore, trans-4-hydroxy-L-proline is able to be produced using proline 4-hydroxylase isolated from a microorganism belonging to the genus *Amycolatopsis* or the genus *Dactylosporangium* (Japanese Published Unexamined Patent Application No. 313179/1995), and cis-3-hydroxy-L-proline is able to be produced using proline 3-hydroxylase isolated from a microorganism belonging to the genus *Streptomyces* (Japanese Published Unexamined Patent Application No. 322885/1995) [*Bioindustry,* 14, 31 (1997)].

A compound where R¹ or R² is acyl is able to be produced from a compound where R¹ or R⁵ is hydrogen by a known method mentioned, for example, in WO 00/51561, etc.

A compound where R² is a saturated or unsaturated hydrocarbon group is able to be produced from a compound where R² is hydrogen by a known method mentioned, for example, in Japanese Published Unexamined Patent Application No. 355531/2000.

When the defined group changes under the condition of the conducted method or is not suitable for conducting the method, an aimed compound is able to be produced by a method for introduction and elimination for protective groups which is commonly used in synthetic organic chemistry [for example, "Protective Groups in Organic Synthesis" by T. W. Greene, published by John Wiley & Sons, Inc. (1981)] and the like.

The resulting compound is able to be purified by a common purifying method such as crystallization and chromatography.

As the pharmaceutically acceptable salt of the compound (I), mention may be made of alkali metal salts such as sodium salts, potassium salts, etc., alkaline earth metal salts such as magnesium salts, calcium salts, etc., ammonium salts such as ammonium, tetramethylammonium, etc., organic amine addition salts to which morpholine, piperidine, etc. and the like.

The lipid metabolism improving agent of the present invention is a pharmaceutical preparation comprising, as an active ingredient, the compound (I) or a salt thereof either solely or in a mixed state or as a mixture with other ingredients for any other treatment and is preferably used as an improving agent for hypertriglyceridemia or hyper-free fatty acidemia.

Such a pharmaceutical preparation is able to be prepared by mixing the active ingredient with one or more pharmaceutically acceptable carriers followed by subjecting to any method which has been well known in the technical field of pharmaceutical preparations.

In administering the preparation, it is desirable to select a route of administration that is the most effective in the treatment and its examples are oral administration and parenteral administrations such as intravenous, intraperitoneal or subcutaneous administration, and an oral administration is preferred.

With regard to the dosage form, any of oral preparation such as tablets, diluted powder, granules, pill, suspensions, emulsion, infusion/decoction, capsules, syrup, liquid, elixir, extract, tincture, fluid extract, etc. and parenteral preparation such as injection, drip infusion, cream, suppository, etc. may be used and an oral preparation is preferably used.

In the manufacture of an oral preparation, it is possible to use additives such as excipient, binder, disintegrating agent, lubricant, dispersing agent, suspending agent, emulsifier, diluting agent, buffer, antioxidant and cell suppressor.

A liquid preparation such syrup which is appropriate for oral administration is able to be prepared by addition of water, saccharide such as sucrose, sorbitol, fructose, etc., glycol such as polyethylene glycol, propylene glycol, etc., oil such as sesame oil, olive oil, soybean oil, etc., antiseptic such as p-hydroxybenzoate, etc., preservative such as p-hydroxybenzoate derivatives (e.g., methyl p-hydroxybenzoate), sodium benzoate, etc., flavor such as strawberry flavor, peppermint, etc. and the like.

Tablets, powder, granule, etc. which are suitable for oral administration are able to be prepared by addition of saccharide such as lactose, sugar, glucose, sucrose, mannitol, sorbitol, etc., starch such as potato, wheat, corn, etc., inorganic substance such as calcium carbonate, calcium sulfate, sodium hydrogen carbonate, sodium chloride, etc., excipient such as crystalline cellulose, plant powder (e.g., powdered licorice and powdered gentian), etc., disintegrating agent such as starch, agar, powdered gelatin, crystalline cellulose, carmellose sodium, carmellose calcium, calcium carbonate, sodium hydrogen carbonate, sodium alginate, etc., lubricant such as magnesium stearate, talc, hydrogenated plant oil, Macrogol, silicone oil, etc. , bonding agent such as polyvinyl alcohol, hydroxypropyl cellulose, methyl cellulose, ethyl cellulose, carmellose, gelatin, starch paste, etc., surfactant such as fatty acid ester, etc., plasticizer such as glycerol, and the like.

Preparation suitable for parenteral administration such as an injection preparation preferably comprises a sterilized aqueous preparation which is isotonic to blood of the person to be administered containing a compound (I) or a salt thereof. For example, in the case of an injection preparation, a solution for injection is prepared using a salt solution, a glucose solution or a carrier comprising a mixture of a salt solution and a glucose solution and the like.

In such a parenteral preparation, it is also possible to add one or more auxiliary component (s) selected from diluent, antiseptic agent, flavor, excipient, lubricant, bonding agent, surfactant, plasticizer, etc. which were exemplified for an oral preparation already.

The dose and the administering frequency of the preparation of the present invention vary depending upon dosage form and age, body weight, nature of symptom to be treated or degree of severeness of a patient and, usually, the preparation is administered once to several times a day so that the dose as the compound (I) or a salt thereof is made 5 mg to 5,000 mg or, preferably, 50 mg to 5,000 mg a day for an adult.

Although there is no particular limitation for the administering period, it is usually from 1 day to 1 year and, preferably, from 2 weeks to 3 months.

The preparation of the present invention is able to be used not only to human being but also to animals except human being (hereinafter, abbreviated as non-human animals).

As the non-human animals, mention may be made of mammals, birds, reptiles, amphibians, fish and animals other than human being.

The dose in the case of administration to non-human animals varies depending upon age and type of the animal and nature or degree of severeness of symptom and, usually, the preparation is administered once to several times a day so that the dose as the compound (I) or a salt thereof is made 0.5 mg to 500 mg or, preferably, 5 mg to 500 mg a day per kg of body weight.

Although there is no particular limitation for the administering period, it is usually from 1 day to 1 year and, preferably, from 2 weeks to 3 months.

By the same method as in the case of the preparation of the present invention, it is possible to prepare a food and drink additives for improvement of lipid metabolism, preferably, the improvement in hypertriglyceridemia or hyper-free fatty acidemia comprising the compound (I) or a salt thereof as an active ingredient.

If necessary, other food and drink additives is mixed with and dissolved in the food and drink additives of the present invention whereupon it is possible to make into the form of, for example, powder, granules, pellets, tablets and various liquid preparations.

The foods and drinks of the present invention are able to be processed and manufactured by the conventional method for the manufacture of foods and drinks except that the compound (I) or a salt thereof or a food and drink additive of the present invention is added to foods and drinks.

The foods and drinks of the present invention are also able to be produced by using granulating methods such as fluidized bed granulation, stirring granulation, extrusion granulation, rolling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method, and extrusion methods using an extruding granulator or an extruder.

The foods and drinks of the present invention may be in any of the forms including juice, refreshing soft drinks, tea, lactic acid beverages, dairy products such as fermented milk, frozen dessert, butter, cheese, yogurt, processed milk and skim milk, meat products such as ham, sausages and hamburger, fish products such as steamed, baked or fried fish paste, egg products such as baked or steamed foods made of beaten eggs, confectionery such as cookies, jellies, chewing gum, candies and snacks, bread, noodles, pickles, smoked foods, dried fish, preserved foods boiled down in soy sauce, salted foods, soups, seasonings, etc.

Furthermore, the foods and drinks of the present invention may take the form of a powdered food, a sheet-shaped food, a bottled food, a canned food, a retort food, a capsule food, a tablet food, a liquid food, a health drink, etc.

The foods and drinks of the present invention are able to be used as a health food and drink or a functional food and drink having an effect for improving lipid metabolism or, preferably, an effect for improving hypertriglyceridemia or hyper-free fatty acidemia.

To the foods and drinks or food and drink additives of the present invention may be added food additives which are commonly used in foods and drinks such as sweeteners, coloring agents, preservatives, thickening stabilizers, antioxidants, color developing agents, bleaching agents, fungicides, gum bases, bittering agents, enzymes, glazing agents, acidulants, seasonings, emulsifiers, nutrient supplements, additional materials for preparation, flavors, spice extracts, etc. which are mentioned, for example, in "Handbook for Indication of Food Additives" (Japan Food Additives Association, published on January 6, 1997).

Adding amount of the compound (I) or a salt thereof or of the food and drink additives to the foods and drinks of the present invention may be appropriately selected depending upon the type of foods and drinks, effect expected by ingestion of said foods and drinks, etc. and, usually, it is added so as to contain 0.1% by weight to 100% by weight or, preferably, 1.0% by weight to 100% by weight therein as the compound (I) or a salt thereof.

Depending upon ingestion form, age and body weight of a person to whom it is ingested, etc., the foods and drinks of the present invention is orally administered or, in other words, ingested once to several times a day so that amount as the compound (I) or a salt thereof is made 5 mg to 5,000 mg or, preferably, 50 mg to 5,000 mg a day to an adult.

Although there is no particular limitation for the ingesting period, it is usually from 1 day to 1 year, preferably, from 2 weeks to 3 months.

By the same method as in the case of the food and drink additives of the present invention, it is possible to prepare a feed additive for improvement of lipid metabolism, preferably, the improvement in hypertriglyceridemia or hyper-free fatty acidemia comprising the compound (I) or a salt thereof as an active ingredient. If necessary, other feed additive is mixed with and dissolved in the feed additives of the present invention whereupon it is possible to make into the form of, for example, powder, granules, pellets, tablets and various liquid preparations.

The feed of the present invention is able to be processed and manufactured by the conventional method for the manufacture of feed except that the compound (I) or a salt thereof or a feed additive of the present invention is added to feed for non-human animals.

The feed for non-human animals include any feed for non-human feed for mammals, birds, reptiles, amphibians, fish, etc. and its examples include feed for pets such as dogs, cats, mice, etc., feed for livestock such as cows, pigs, etc., feed for poultry such as hens, turk, etc. and feed for cultivated fish such as sea breams, young yellowtails, etc., and the like.

Examples of the feed to which the compound (I) or a salt thereof or the feed additive of the present invention is to be added include cereals, chaff and bran, vegetable oil cakes, animal-based feed materials, other feed materials, purified products thereof, etc.

As the cereals, mention may be made of milo, wheat, barley, oats, rye, brown rice, buckwheat, foxtail millet, broomcorn millet, Japanese millet, corn, soybean, etc.

As the chaff and bran, mention may be made of rice bran, defatted rice bran, wheat bran, wheat middlings, wheat germ, barley bran, pellet, corn bran, corn germ, etc.

As the vegetable oil cakes, mention may be made of soybean oil cake, soybean flour, linseed oil cake, cottonseed oil cake, peanut oil cake, safflower oil cake, coconut oil cake, palm oil cake, sesame oil cake, sunflower oil cake, rapeseed oil cake, kapok oil cake, mustard seed oil cake, etc.

As the animal-based feed materials, mention may be made of fish powder (such as northern ocean meal, imported meal, whole meal and coastal meal), fish soluble, meat powder, meat and bone powder, blood powder, degraded hair, bone powder, treated by-products for livestock, feather meal, silkworm pupa, skim milk, casein, dry whey, etc.

As other feed materials, mention may be made of stalks and leaves of plants (such as alfalfa, hay cube, alfalfa leaf meal, powder of false acacia, etc.), by-products from the corn processing industry (such as corn gluten meal, corn gluten feed, corn steep liquor, etc.), processed starch products (such as starch, etc.), sugar, products from the fermentation industry (such as yeast, beer cake, malt root, alcohol cake, soy sauce cake, etc.), agricultural by-products (such as processed citrus fruit cake, tofu cake, coffee cake, cocoa cake, etc.), cassava, broad bean, guar meal, seaweeds, krill, spirulina, chlorella, minerals, etc.

As the purified products thereof, mention may be made of proteins (such as casein, albumin, etc.), amino acids, saccharides (such as starch, cellulose, sucrose, glucose, etc.), minerals, vitamins, etc.

The feed of the present invention is also to be produced by using granulating methods such as fluidized bed granulation, stirring granulation, extrusion granulation, tumbling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method and extrusion methods using an extruding granulator or an extruder.

The feed of the present invention is able to be used as a feed for improving lipid metabolism and, preferably, as a feed for improving hypertriglyceridemia or hyper-free fatty acidemia.

Adding amount of the compound (I) or a salt thereof or of the feed additive to the feed of the present invention may be appropriately selected depending upon the type of feed, effect expected by ingestion of said feed, etc. and, usually, it is added so as to contain 0.1% by weight to 100% by weight or, preferably, 1.0% by weight to 100% by weight therein as the compound (I) or a salt thereof.

When the feed of the present invention is ingested to non-human animals, depending upon ingestion form, type of the ingesting animals, age and body weight of the animal, etc., the feed is orally administered or, in other words, ingested once to several times a day so that amount as the compound (I) or a salt thereof is made 0.5 mg to 500 mg or, preferably, 5 mg to 500 mg a day to an adult.

Although there is no particular limitation for the ingesting period, it is usually from 1 day to 1 year and, preferably, from 2 weeks to 3 months.

When the compound (I) or a salt thereof is administered to human being or non-human animals by the above-mentioned method, it is possible to improve lipid metabolism, preferably, improve hypertriglyceridemia or hyper-free fatty acidemia in the human being or non-human animals.

### Best Mode for Conducting the Invention

### Example 1

KK-Ay/Ta Jcl mice (CLEA JAPAN; males; six weeks age) (25 mice) which are animal models for appearing hypertriglyceridemia and hyper-free fatty acidemia were divided into five groups each comprising five mice and named group 1 to group 5.

The mice of group 1 to group 5 were made free to take feed and water. A commercially available feed CE-2 (manufactured by CLEA JAPAN) was ingested to the mice of group 1. CE-2 to which 1% by weight of trans-4-hydroxy-L-proline (manufactured by Kyowa Hakko Kogyo; hereinafter, abbreviated as hydroxyproline) was added was ingested to the mice of group 2. CE-2 to which 1% by weight of trans-N-acetyl-4-hydroxy-L-proline (manufactured by Kyowa Hakko Kogyo; hereinafter, abbreviated as N-acetylhydroxyproline) was added was ingested to the mice of group 3. CE-2 to which 1% by weight of trans-4-hydroxy-L-proline ethyl ester (manufactured by Sanyo Kagaku Kenkyusho; hereinafter, abbreviated as hydroxyproline ethyl ester) was added was ingested to the mice of group 4. CE-2 to which 1% by weight of trans-N,O-diacetyl-4-hydroxy-L-proline oleyl ester (manufactured by Nissei Kagaku; hereinafter, abbreviated as diacetylhydroxyproline oleyl ester) was added was ingested to the mice of group 5.

The mice were fasted for 18 hours during the 10th day to the 11th day after the start of the test and whole blood was collected from descending vena carva to prepare serum. Concentration of triglyceride in the serum was measured by a Triglyceride G-Test Wako (manufactured by Wako Pure Chemical) and concentration of free fatty acids therein was measured by an NEFA C-Test Wako (manufactured by Wako Pure Chemical). The value was shown by mean value ± standard error (n = 5) and statistic ratio of risk (p value) was determined by a t-test. With regard to the ingested amounts during the test period, there was no significant difference among the groups 1 to 5.

Results of measurement of serum triglyceride are shown in Table 1.

**Table 1**

| | Triglyceride (mg/dl) |
|---|---|
| Group 1 | 282.4 ± 30.3 |
| Group 2 | 224.3 ± 23.1 |
| Group 3 | 156.4 ± 23.3* |
| Group 4 | 168.7 ± 13.4* |
| Group 5 | 217.6 ± 18.3 |

| | |
|---|---|
| (*: p < 0.01, to Group 1) | |

It is apparent from Table 1 that, as compared with the serum triglyceride concentration in Group 1, serum triglyceride concentrations in Group 3 and Group 4 were significantly low. Serum triglyceride concentrations in Group 2 and Group 5 also tended to show low values as compared with the serum triglyceride concentration in Group 1. From the result, it is now apparent that hydroxyproline, N-acetylhydroxyproline, hydroxyproline ethyl ester and diacetylhydroxyproline oleyl ester have an action for improving hypertriglyceridemia.

Results of measurement of free fatty acid concentrations in serum are shown in Table 2.

**Table 2**

| | Free Fatty Acid (mEq/1) |
|---|---|
| Group 1 | 1.12 ± 0.05 |
| Group 2 | 0.84 ± 0.05* |
| Group 3 | 0.77 ± 0.06* |
| Group 4 | 0.68 ± 0.02* |
| Group 5 | 1.00 ± 0.07 |

| | |
|---|---|
| (*: p < 0.005, to Group 1) | |

It is apparent from Table 2 that, as compared with the free fatty acid concentration in serum in Group 1, free fatty acid concentrations in serum in Group 2, Group 3 and Group 4 were significantly low. Free fatty acid concentration in serum in Group 5 also tended to show low values as compared with the free fatty acid concentration in serum in Group 1. From the result, it is apparent that hydroxyproline, N-acetylhydroxyproline, hydroxyproline ethyl ester and diacetylhydroxyproline oleyl ester have an action for improving hyper-free fatty acidemia.

### Example 2

Water was added to a composition having the following formulation mentioned in Table 3 to make 1,000 ml whereupon a refreshing soft drink (for ten bottles) for improvement of lipid metabolism was prepared.

**Table 3**

| Composition | Amount |
|---|---|
| Hydroxyproline | 5 g |
| Vitamin C | 1 g |
| Vitamin B₁ | 5 mg |
| Vitamin B₂ | 10 mg |
| Vitamin B₆ | 25 mg |
| Liquid Sugar | 150 g |
| Citric Acid | 3 g |
| Flavor | 1 g |

### Example 3

A composition having the formulation mentioned in Table 4 was extracted with 1,000 ml of water to prepare 1,000 ml of tea beverage for improvement of lipid metabolism.

**Table 4**

| Composition | Amount |
|---|---|
| Hydroxyproline | 5 g |
| Tea Leaves | 15 g |

### Example 4

According to the formulation mentioned in Table 5, chewing gum (for 30 pieces) for improvement of lipid metabolism was prepared.

**Table 5**

| Composition | Amount |
|---|---|
| Hydroxyproline | 1.5 g |
| Gum Base | 25 g |

| | |
|---|---|
| Sugar | 63 g |
| Starch Syrup | 10 g |
| Flavor | 1 g |

### Example 5

According to the formulation mentioned in Table 6, candy (for 20 products) for improvement of lipid metabolism was prepared.

**Table 6**

| Composition | Amount |
|---|---|
| Hydroxyproline | 1 g |
| Sugar | 80 g |
| Starch Syrup | 20 g |
| Flavor | 0.1 g |

### Example 6

According to the formulation mentioned in Table 7, tablets (200 mg per tablet) for improvement of lipid metabolism were prepared by a conventional method.

**Table 7**

| Composition | Amount |
|---|---|
| N-Acetylhydroxyproline | 50 mg |
| Lactose | 90 mg |
| Corn Starch | 30 mg |
| Synthetic Aluminum Silicate | 12 mg |
| Carboxymethylcellulose Calcium | 15 mg |
| Magnesium Stearate | 3 mg |

### Example 7

According to the formulation mentioned in Table 8, a powder (550 mg per chartula) for improvement of lipid metabolism was prepared.

**Table 8**

| Composition | Amount |
|---|---|
| Hydroxyproline Methyl Ester | 50 mg |
| Lactose | 300 mg |
| Corn Starch | 200 mg |

### Example 8

According to the formulation mentioned in Table 9, a hard capsule preparation (160 mg per capsule) for improvement of lipid metabolism was prepared.

**Table 9**

| Composition | Amount |
|---|---|
| Hydroxyproline Methyl Ester | 50 mg |
| Lactose | 60 mg |
| Corn Starch | 30 mg |
| Hydroxypropyl Cellulose | 20 mg |

To 50 mg of hydroxyproline ethyl ester were added 60 mg of lactose and 30 mg of corn starch, and mixing was carried out. An aqueous solution of 20 mg of hydroxypropyl cellulose was added thereto and the mixture was kneaded. Then, granules were prepared using an extruding granulator. The granules were filled in gelatin hard capsules to prepare a hard capsule preparation.

### Example 9

According to the formulation mentioned in Table 10, a soft capsule preparation (170 mg per capsule) for improvement of lipid metabolism was prepared.

**Table 10**

| Composition | Amount |
|---|---|
| Diacetylhydroxyproline Oleyl Ester | 50 mg |
| Soybean Oil | 120 mg |

To 120 mg of soybean oil was added 50 mg of diacetylhydroxyproline oleyl ester, and mixing was carried out. Then, the mixture was filled in soft capsules using an automated molding machine of a rotary dies type by a conventional method whereupon a soft capsule preparation was prepared.

### Industrial Applicability

In accordance with the present invention, there is provided a lipid metabolism improving agent comprising hydroxyproline, hydroxyproline derivative or a pharmaceutically acceptable salt thereof as an active ingredient or a food and drink, a food and drink additive, a feed or a feed additive for improvement of lipid metabolism comprising the same.

## Claims

1. A lipid metabolism improving agent, which comprises, as an active ingredient, hydroxyproline or a hydroxyproline derivative represented by the formula (I) [hereinafter referred to as the compound (I)] or a pharmaceutically acceptable salt thereof: 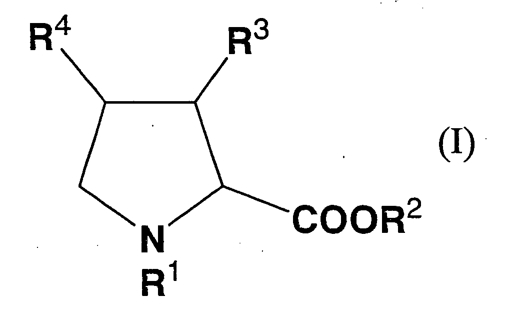 [wherein R¹ is hydrogen or acyl; R² is hydrogen or a saturated or unsaturated hydrocarbon group; and one of R³and R⁴is hydrogen while the other is OR⁵ (in which R⁵ is hydrogen or acyl)].

2. The lipid metabolism improving agent according to claim 1, wherein the lipid metabolism improvement is intended for improvement of hypertriglyceridemia or a hyper-free fatty acidemia.

3. A food and drink or food and drink additive for improvement of lipid metabolism, which comprises the compound (I) or a pharmaceutically acceptable salt thereof as an effective ingredient.

4. The food and drink or food and drink additive for improvement of lipid metabolism according to claim 3, wherein the lipid metabolism improvement is intended for improvement of hypertriglyceridemia or a hyper-free fatty acidemia.

5. A feed or feed additive for improvement of lipid metabolism, which comprises the compound (I) or a pharmaceutically acceptable salt thereof as an active ingredient.

6. The feed or feed additive for improvement of lipid metabolism according to claim 5, wherein the lipid metabolism improvement is intended for improvement of hypertriglyceridemia or a hyper-free fatty acidemia.

7. A method for improving lipid metabolism, which comprises administering the compound (I) or a pharmaceutically acceptable salt thereof.

8. The method according to claim 7, wherein the lipid metabolism improvement is intended for improvement of hypertriglyceridemia or a hyper-free fatty acidemia.

9. Use of the compound (I) or a pharmaceutically acceptable salt thereof for the manufacture of a lipid metabolism improving agent.

10. Use of the compound (I) or a pharmaceutically acceptable salt thereof for the manufacture of foods and drinks or food and drink additives for improvement of lipid metabolism.

11. Use of the compound (I) or a pharmaceutically acceptable salt thereof for the manufacture of feeds or feed additives for improvement of lipid metabolism.
